(19) Europäisches Patentamt
European Patent Office
Office européen des brevets

(11) Veröffentlichungsnummer: **0 370 946 A2**

# (12) EUROPÄISCHE PATENTANMELDUNG

(21) Anmeldenummer: **89810847.7**

(22) Anmeldetag: **08.11.89**

(51) Int. Cl.5: **C08F 8/08, C08F 16/16, C07D 301/12, C07D 303/20, C07D 303/22, C07D 303/27**

(30) Priorität: **17.11.88 CH 4260/88**

(43) Veröffentlichungstag der Anmeldung: **30.05.90 Patentblatt 90/22**

(84) Benannte Vertragsstaaten: **CH DE ES FR GB IT LI NL**

(71) Anmelder: **CIBA-GEIGY AG**
**Klybeckstrasse 141**
**CH-4002 Basel(CH)**

(72) Erfinder: **Wolleb, Heinz, Dr.**
**Les Arneys**
**CH-1731 Ependes(CH)**

## (54) Verfahren zur Herstellung von Epoxidverbindungen.

(57) Beschrieben wird ein Verfahren zur Herstellung von Epoxidverbindungen durch Epoxidierung von aromatischen Allylethern mit Dioxiranen. Mit dem Verfahren lassen sich in hoher Ausbeute halogenarme Epoxidharze mit hohen Epoxidwerten herstellen.

EP 0 370 946 A2

## Verfahren zur Herstellung von Epoxidverbindungen

Die vorliegende Erfindung betrifft ein verbessertes Verfahren zur Herstellung von Epoxidverbindungen, sowie die Herstellung gehärteter Produkte aus diesen Verbindungen.

Epoxidharze werden seit langem unter anderem in der Elektronikindustrie verwendet. In den letzten Jahren sind die Anforderungen an die Reinheit dieser Harze ständig gewachsen. Insbesondere können geringe Mengen an ionischem oder hydrolysierbarem Halogen störend wirken. Auch werden zunehmend Harze mit einem möglichst hohen Epoxidierungsgrad verlangt. In der Regel werden Epoxidharze durch Umsetzung von Verbindungen mit reaktiven Wasserstoffatomen, vorzugsweise Phenolen, mit Epichlorhydrin hergestellt. Zur Herstellung halogenarmer Harze sind auch Verfahren bekannt, bei denen Verbindungen mit ethylenisch ungesättigten Bindungen, epoxidiert werden. Im Falle der Allylverbindungen handelt es sich dabei in der Regel um aromatische C-Allylverbindungen, die mit Persäuren, beispielsweise mit Peressigsäure, epoxidiert werden. Beispiele für solche Umsetzungen sind in der EP-A-205,402 zu finden.

Die Epoxidierung von ethylenisch ungesättigten Verbindungen mit Dimethyldioxiran der Formel 1 ist an sich bekannt

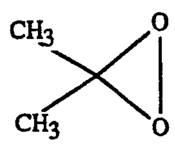

(I).

So beschreiben beispielsweise P.F. Corey et al. in J. Org. Chem., **51**, 1925-6 (1986) die Umsetzung von $\alpha,\beta$-ungesättigten Carbonsäuren, unter anderem von Zimtsäure, mit Dimethyldioxiran. Ferner beschreiben G. Cicala et al. in J. Org. Chem., **47** ,2670-3 (1982) die Umsetzung von aliphatischen Allylalkoholen mit Dimethyldioxiran als Epoxidierungsreagenz.

Die Epoxidierung von aromatischen Allylethern mit Persäuren ist beispielsweise in der US-A-3,957,873 beschrieben. Bei dieser Umsetzung hat sich gezeigt, dass die Reaktion in der Regel nur mit mässigen Ausbeuten und Epoxidgehalten verläuft. Ausserdem stellt die Verwendung von Persäuren bei grosstechnischen Verfahren ein Sicherheitsrisiko dar.

Es wurde jetzt überraschenderweise gefunden, dass sich bei der Epoxidierung von aromatischen Allylethern hohe Ausbeuten und hohe Epoxidgehalte erhalten lassen, wenn die Umsetzung mit Dioxiranen durchgeführt wird. Ausserdem lässt sich das Sicherheitsrisiko bei solchen Epoxidierungen im Vergleich zum Einsatz von Persäuren verringern.

Die vorliegende Erfindung betrifft ein Verfahren zur Herstellung von Epoxidverbindungen aus aromatischen Allylethern mit mindestens einem direkt an den aromatischen Kern gebundenem Rest der Formel II

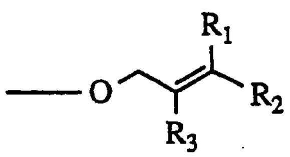

(II),

worin $R_1$, $R_2$ und $R_3$ unabhängig voneinander Wasserstoff oder $C_1$-$C_6$Alkyl, insbesondere Methyl und ganz besonders Wasserstoff bedeuten, dadurch gekennzeichnet, dass man das Ausgangsprodukt als solches oder in einem inerten Lösungsmittel gelöst mit einem Dioxiran epoxidiert, wobei auf eine Allylgruppe etwa 1 bis 20 Mole Dioxiran eingesetzt werden.

Zu den bevorzugten im erfindungsgemässen Verfahren einzusetzenden aromatischen Allylethern zählen die Verbindungen der Formeln III oder Verbindungen, die im wesentlichen aus wiederkehrenden Struktureinheiten der Formeln IV oder V bestehen

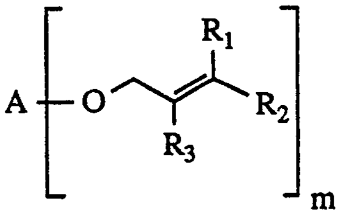 (III),

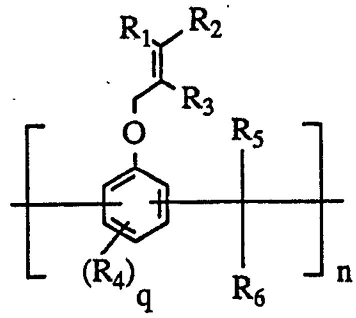 (IV),

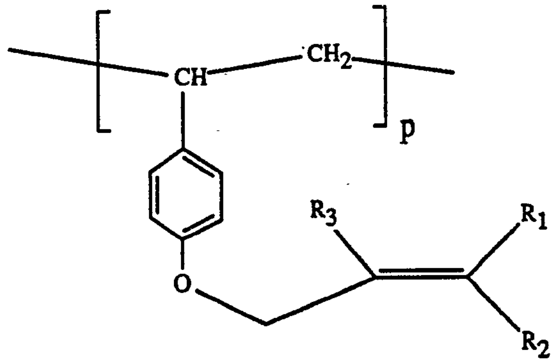 (V),

worin m 1, 2,3 oder4 ist, n eine ganze Zahl von 2 bis 12 ist, p eine ganze Zahl von 2 bis 100 bedeutet, q 0,1,2 oder 3 ist, $R_1$, $R_2$ und $R_3$ die oben definierte Bedeutung besitzen, $R_4$ $C_1$-$C_6$Alkyl oder Halogen bedeutet, $R_5$ und $R_6$ unabhängig voneinander Wasserstoff oder $C_1$-$C_6$Alkyl sind und A einen m-wertigen aromatischen Rest eines Phenols nach dem Entfernen der Hydroxylgruppen darstellt.

Bedeuten irgendwelche Reste $C_1$-$C_6$Alkyl, so handelt es sich dabei um verzweigte oder vorzugsweise um geradkettige Alkylreste. Beispiele dafür sind Methyl, Ethyl, n-Propyl, Isopropyl, n-Butyl, sek.Butyl, tert.Butyl, n-Pentyl oder n-Hexyl.

Bevorzugt werden Ethyl, n-Propyl oder n-Butyl, insbesondere jedoch Methyl.

Der Index m ist vorzugsweise 2.

Der Index n ist vorzugsweise 3 bis 6 und der Index p ist vorzugsweise 5 bis 40.

Der Index q ist vorzugsweise 1 und ganz besonders bevorzugt 0.

$R_4$ bedeutet als Halogen in der Regel Chlor oder Brom.

Beispiele für bevorzugte Verbindungen, von denen sich die im erfindungsgemässen Verfahren einzusetzenden Allylether ableiten, sind ein- oder mehrkernige Monophenole, wie Phenol, Kresole oder Chlorphenol, oder insbesondere ein- oder mehrkernige Polyphenole, wie Resorcin, Hydrochinon, Bis-(hydroxyphenyl)-methan (Bisphenol F), 2,2,-Bis-(4-hydroxyphenyl)-propan (Bisphenol A), bromiertes 2,2,-Bis-(4-hydroxyphenyl)-propan, Bis-(4-hydroxyphenyl)-ether, Bis-(4-hydroxyphenyl)-sulfon, 1,3,5-Trihydroxybenzol, 1,1,2,2-Tetrakis-(4-hydroxyphenyl)-ethan, Novolake, die durch Kondensation von Aldehyden, wie Formaldehyd, Acetaldehyd, Chloral oder Furfuraldehyd mit gegebenenfalls alkyl- oder halogen-substituierten Phenolen, wie Phenol, den oben beschriebenen Bisphenolen, 2- oder 4-Methylphenol, 4-tert.Butylphenol, p-Nonylphenol oder 4- Chlorphenol erhältlich sind, oder Polyvinylphenole, wie Poly-p-vinylphenol.

Die Ausgangsallylether lassen sich aus diesen Mono- oder Polyphenolen durch Veretherung mit einem Allylhalogenid, insbesondere mit Allylchlorid, in an sich bekannter Weise herstellen.

Bevorzugte Ausgangsmaterialien sind Verbindungen der Formeln III, IV oder IV, worin $R_1$ und $R_2$ Wasserstoff bedeuten und $R_3$ Methyl oder insbesondere Wasserstoff ist.

Ganz besonders bevorzugte Ausgangsmaterialien sind Verbindungen der Formeln III, IV oder V, worin m 2 ist, n 3 bis 6 bedeutet, p 5 bis 40 darstellt, q 0 ist, A ein zweikerniger Rest eines Bisphenols ist und $R_1$, $R_2$ und $R_3$ Wasserstoff bedeuten.

Das Ausgangsmaterial kann sowohl mit einer Lösung eines Dioxirans in einem Keton epoxidiert werden oder das Dioxiran wird in situ im Reaktionsgemisch mit dem Ausgangsmaterial hergestellt.

Die Herstellung von Dioxiranlösungen ist an sich bekannt und wird beispielsweise von W. Adam et al. in J. Org. Chem., 52, 2800-3 (1987) oder von R. Mello in J. Org. Chem., 53, 3890-1 (1988) beschrieben. Dazu wird ein Peroxomonosulfat, beispielsweise Kaliumperoxomonosulfat, zu einer Lösung aus einem Keton, insbesondere einem aliphatischen Keton, wie Aceton, Hexafluoraceton, Methylethylketon oder Cyclohexanon gegeben, welche mit einem Puffer auf einen pH-Wert von etwa 6 bis 10, insbesondere 6 bis 8, eingestellt

ist. Das entstandene Dioxiran kann gegebenenfalls konzentriert werden. Die Lösung kann als solche direkt zur Epoxidierung verwendet werden oder unter Kühlung einige Zeit gelagert werden. Als Peroxomonosulfate lassen sich beispielsweise die Alkalimetallsalze der Peroxomonoschwefelsäure (Carotsche Säure), wie die Natrium-oder Kaliumsalze, einsetzen. So lässt sich für diesen Zweck beispielsweise das Produkt Oxone® der Fa. Du Pont verwenden. Dabei handelt es sich um ein Gemisch aus Kaliumperoxomonosulfat mit Kaliumsulfat und Kaliumhydrogensulfat.

Die Epoxidierung von aromatischen Allylethern mit Dioxiranlösungen lässt sich beispielsweise wie folgt ausführen:

Das Ausgangsprodukt wird als solches oder in einem unter den Reaktionsbedingungen inerten Lösungsmittel vorgelegt und die Dioxiranlösung im entsprechenden Keton zugegeben. Pro Mol zu epoxidierender Doppelbindung werden in der Regel etwa ein bis zwanzig Mole Dioxiran eingesetzt. Die Zugabe der Dioxiranlösung und die anschliessende Umsetzung können unter Kühlung oder unter Erhitzen bis zum Rückfluss des Reaktionsgemisches erfolgen. Die Epoxidierung dauert, je nach Reaktivität der Reaktanden und der angewendeten Temperaturen, im allgemeinen 0,5 bis 24 Stunden.

Die Epoxidierung des aromatischen Allylethers, wobei das Dioxiran in situ im Reaktionsgemisch mit dem Ausgangsprodukt hergestellt wird, lässt sich beispielsweise wie folgt ausführen:

Das Ausgangsprodukt wird als solches oder in einem unter den Reaktionsbedingungen inerten Lösungsmittel zusammen mit einem Phasentransferkatalysator und in Kombination mit einem Keton, insbesondere einem aliphatischen Keton, wie Aceton, Hexafluoraceton, Methylethylketon oder Cyclohexanon, und Wasser vorgelegt, so dass ein Zweiphasensystem entsteht. Die wässrige Phase enthält einen Puffer, mit dem ein pH-Wert im Bereich von etwa 6 bis 10, insbesondere 6 bis 8, eingestellt wird. Anschliessend wird ein Peroxomonosulfat, beispielsweise Oxone®, zugegeben. Die Menge an Peroxomonosulfat beträgt im allgemeinen etwa ein bis zehn Mol pro Mol zu epoxidierender Doppelbindung. Das Peroxomonosulfat kann als Feststoff oder gelöst in gepuffertem Wasser (pH-Wert etwa 6 bis 10, je nach pH-Wert im Zweiphasensystem) zugesetzt werden. Während der Zugabe empfiehlt es sich, den pH-Wert des Reaktionsgemisches zu kontrollieren und gegebenenfalls durch Zugabe einer Base, wie Natronlage oder Kalilauge, konstant zu halten. Die Epoxidiervng dauert, je nach Reaktivität der Reaktanden und der angewendeten Temperaturen, im allgemeinen 0,5 bis 24 Stunden.

In dieser Variante lassen sich alle an sich bekannten Phasentransferkatalysatoren einsetzen. Insbesondere verwendet man Kronenether, wie 18-Krone-6, oder quaternäre Ammonium- oder Phosphoniumsalze, wie Tetrabutylammoniumhydrogensulfat oder Tetrabutylphosphoniumhydrogensulfat.

Als Pufferlösungen lassen sich alle an sich üblichen Puffer einsetzen. Bevorzugt verwendet man Phosphatpuffer, mit denen ein pH-Wert von etwa 7 eingestellt wird.

Beispiele für unter den Reaktionsbedingungen inerte Lösungsmittel für das Ausgangsmaterial sind aromatische Kohlenwasserstoffe, die gegebenenfalls halogeniert sein können, wie Benzol, Toluol, Xylole, Cumol, Chlorbenzol oder Dichlorbenzol, oder halogenierte aliphatische Kohlenwasserstoffe, wie Trichlorethan, Tetrachlorethan, Dichlormethan oder Chloroform, oder Ester, wie beispielsweise Essigsäureethylester.

Das erfindungsgemässe Verfahren kann an der Luft oder unter Schutzgas, beispielsweise unter Stickstoff oder Argon, durchgeführt werden.

Bevorzugte Reaktionstemperaturen liegen zwischen 0 und 30 °C.

Besonders bevorzugte Epoxidierungsreagenzien sind Bis-trifluormethyl-dioxiran oder insbesondere Dimethyldioxiran.

In einer besonders bevorzugten Ausführungsform des erfindungsgemässen Verfahrens reinigt man den aromatischen Allylether vor der Epoxidierungsstufe, beispielsweise durch Chromatographie oder insbesondere durch Destillation; im Falle von höhermolekularen Ausgangsprodukten lässt sich das Ausgangsmaterial durch Dünnschicht- oder Kurzwegdestillation reinigen. Diese Verfahrensvariante führt im allgemeinen zu besonders hohen Ausbeuten und Epoxidwerten.

In einer weiteren besonders bevorzugten Ausführungsform des erfindungsgemässen Verfahrens führt man die Epoxidierung wie oben beschrieben in einem Zweiphasensystem enthaltend einen aromatischen Allylether gelöst in einem inerten Lösungsmittel und eine wässrige Ketonphase durch und stellt das Dioxiran in situ im Reaktionsgemisch her, wobei das Peroxomonosulfat fest zugegeben wird und der pH-Wert durch Zugabe einer wässrigen Lösung einer Base, insbesondere Natronlauge, konstant gehalten wird.

Bei dieser Ausführungsform beträgt der pH-Wert vorzugsweise 6,5 bis 7,5, der aromatische Allylether wird in einem inerten Lösungsmittel zusammen mit einem Kronenether oder einem quaternären Ammoniumsalz als Phasentransferkatalysator vorgelegt und als Keton verwendet man Aceton.

Die erfindungsgemäss erhältlichen Harze lassen sich in an sich üblicher Weise durch Zugabe eines Härtungsmittels, beispielsweise eines primären oder sekundären Polyamins, eines Anhydrids einer Polycarbonsäure oder eines katalytisch wirkenden Härtungsmittels, wie eines tertiären Amins, und gegebenenfalls

durch Erhitzen der Zusammensetzung härten. Die gehärteten Produkte zeichnen sich insbesondere durch einen geringen Halogengehalt aus.

Die Erfindung betrifft auch ein Verfahren zur Herstellung von gehärteten Produkten, dadurch gekennzeichnet, dass man die nach dem erfindungsgemässen Verfahren erhältlichen Epoxidharze durch Zugabe eines an sich bekannten Härtungsmittels und gegebenenfalls durch Erhitzen härtet.

Die härtbaren Gemischen können gewünschtenfalls weitere an sich übliche Zusätze, wie reaktive Verdünner, Weichmacher, Streck-, Füll- und Verstärkungsmittel, beispielsweise Steinkohlenteer, Bitumen, Textilfasern, Glasfasern, Kohlenstoff-Fasern, mineralische Silikate, Glimmer, Quarzmehl, Aluminiumoxidhydrat, Bentonite, Wollastonit, Kaolin, Kieselsäureaerogel oder Metallpulver, wie Aluminiumpulver oder Eisenpulver, ferner Pigmente und Farbstoffe, wie Russ, Oxidfarben und Titandioxid, sowie Flammschutzmittel, Thixotropiemittel, Verlaufmittel (die zum Teil auch als Formtrennmittel Anwendung finden), wie Silicone, Wachse und Stearate, oder Haftvermittler, Antioxidantien und Lichtschutzmittel enthalten.

Die erfindungsgemässen Gemische lassen sich ganz allgemein zur Herstellung von gehärteten Produkten einsetzen, und können in der dem jeweils speziellen Anwendungsgebiet angepassten Formulierung, in ungefülltem oder gefülltem Zustand als Komponenten von Anstrichmitteln, Beschichtungsmassen, Lacken, Pressmassen, Tauchharzen, Wirbelsinterpulvern, Spritzgussformulierungen, Werkzeugharzen, Pulverlacken, Giessharzen, Imprägnierharzen, Laminierharzen, Klebmitteln oder Matrixharzen eingesetzt werden.

Die erfindungsgemäss erhältlichen Harze eignen sich beispielsweise für den Einsatz auf den Gebieten des Oberflächenschutzes, der Laminierverfahren, im Bauwesen und insbesondere in der Elektrotechnik und der Elektronik. Verbindungen mit durchschnittlich einer Epoxidgruppe lassen sich als reaktive Verdünner einsetzen.

## Beispiele

### Beispiel 1: Herstellung von Bisphenol-A-Diglycidylether

Zu 15,4 g (0,05 Mol) destilliertem Bisphenol-A-Diallylether (hergestellt nach der DE-A-26 27 045) und 0,66 g 18-Krone-6 in einem Zweiphasensystem von 50 ml Aceton, 50 ml Dichlormethan und 100 ml Phosphatpuffer pH 7,0 werden innerhalb von dreieinhalb Stunden unter starkem Rühren bei Raumtemperatur 50 g (0,08 Mol) festes Oxone® gegeben, wobei der pH mit 15 %iger NaOH-Lösung konstant gehalten wird. Nach weiteren 15 Minuten werden die Phasen getrennt, die organische Phase mit 10 ml Aceton, 5 ml Dichlormethan, 100 ml Phosphatpuffer pH 7,0 und 0,66 g 18-Krone-6 versetzt und innerhalb von dreieinhalb Stunden weitere 50 g (0,08 Mol) festes Oxone® zugegeben. Nach 15 Minuten werden die Phasen wieder getrennt und die oben beschriebenen Operationen noch einmal durchgeführt, wobei 30 g (0,05 Mol) Oxone® verwendet werden. Anschliessend wird das Reaktionsgemisch zweimal mit 100 ml Toluol extrahiert, die vereinigten organischen Phasen am Rotavapor auf 200 ml eingeengt. Die Toluolphase wird dreimal mit 50 ml 1N NaOH-Lösung und fünfmal mit 50 ml Wasser gewaschen, über $MgSO_4$ getrocknet, filtriert und eingedampft. Man erhält 13,6 g (80 %) einer goldgelben Flüssigkeit mit folgenden Eigenschaften:

Epoxidzahl: 5,11 Val/kg (87 %); $\eta^{25}$: 3730 mPa.s;
totaler Chlorgehalt: 425 ppm; hydrolisierbarer Chlorgehalt: 128 ppm.

### Beispiel 2: Herstellung von Bisphenol-A-Diglycidylether

Zu 0,31 g (1 mMol) destilliertem Bisphenol-A-Diallylether werden unter Stickstoffatmosphäre bei Raumtemperatur 87 ml einer 0,1 M Lösung von Dimethyldioxiran in Aceton [hergestellt nach W. Adam et al., J. Org. Chem, **52**,2800-3 (1987)] gegeben und sechs Stunden gerührt. Anschliessend werden das Lösungsmittel und das überschüssige Dimethyldioxiran am Rotavapor abdestilliert. Man erhält 0,33 g (97 %) einer leicht gelben, klaren Flüssigkeit mit einer Epoxidzahl von 4,8 Val/kg (82 %).

### Beispiel 3: Herstellung von Bisphenol-A-di-(2,3-epoxibutyl)-ether

Zu 3,36 g (0,01 Mol) destilliertem Bisphenol-A-dicrotylether (hergestellt analog zum Diallylether nach

der DE-A-26 27 045) und 0,6 g 18-Krone-6 in einem Zweiphasensystem von 50 ml Aceton, 50 ml Dichlormethan und 50 ml Phosphatpuffer pH 7,0 werden innerhalb von eineinviertel Stunden bei Raumtemperatur 23 g(0,04 Mol) Oxone® gelöst in 100 ml Phosphatpuffer pH 7,0 getropft, wobei der pH mit 15 %iger NaOH-Lösung konstant gehalten wird. Danach wird noch weitere eineinhalb Stunden gerührt und dann das Reaktionsgemisch zweimal mit 100 ml Dichlormethan extrahiert. Die vereinigten organischen Phasen werden zweimal mit 50 ml Wasser gewaschen, über Natriumsulfit peroxidfrei gemacht, über $MgSO_4$ getrocknet, filtriert und eingedampft. Man erhält 3,5 g (95 %) einer klaren, hellbraunen Flüssigkeit mit einer Epoxidzahl von 5,05 Val/kg (93 %).

Beispiel 4: **Herstellung von Bisphenol-A-di-(2,3-epoxibutyl)-ether**

Zu 0,17 g (0,5 mMol) destilliertem Bisphenol-A-dicrotylether werden unter Stickstoffatmosphäre bei Raumtemperatur 33 ml einer 0,15 M Lösung von Dimethyldioxiran in Aceton gegeben und eindreiviertel Stunden gerührt. Anschliessend werden das Lösungsmittel und das überschüssige Dimethyldioxiran am Rotavapor abdestilliert. Man erhält 0,17 g (92 %) einer leicht gelben, klaren Flüssigkeit mit einer Epoxidzahl von 4,9 Val/kg (91 %).

Beispiel 5: **Herstellung von Poly-4-(2,3-epoxi-butyloxi)-styrol**

a)Herstellung von Poly-4-(2,3-butenoxy)-styrol

Zu einer Lösung von 60,1 g Resin® M (Poly-p-vinylphenol; Maruzen Oil) und 128,3 g Crotylchlorid in 300 ml DMSO werden innerhalb von 10 Minuten bei Raumtemperatur unter Rühren 107,7 g $K_2CO_3$ gegeben und anschliessend viereinhalb Stunden bei 60 °C gerührt. Danach wird das Reaktionsgemisch abgekühlt, mit 300 ml Wasser versetzt und dreimal mit 100 ml Essigsäureethylester extrahiert. Die vereinigten organischen Phasen werden dreimal mit 100 ml Wasser gewaschen, über $MgSO_4$ getrocknet, filtriert und eingedampft. Man erhält 82,8 g (95 %) eines braunen Harzes. $\eta^{120}$ = 1560 mPa.s.

b)Herstellung von Poly-4-(2,3-epoxi-butyloxi)-styrol

Zu 8,7 g des Polycrotylethers aus Beispiel 5a) und 0,6 g 18-Krone-6 in einem Zweiphasensystem von 50 ml Aceton, 50 ml Dichlormethan und 100 ml Phosphatpuffer pH 7,0 werden unter starkem Rühren innerhalb von dreieinviertel Stunden bei Raumtemperatur 90 g (0,15 Mol) Oxone® gegeben, wobei der pH-Wert mit 15 %iger NaOH-Lösung konstant gehalten wird. Die Suspension wird danach noch eine Stunde gerührt und dann filtriert. Das Filtrat wird dreimal mit 100 ml Methylethylketon extrahiert, die vereinigten organischen Phasen zweimal mit 100 ml gesättigter NaCl-Lösung gewaschen, mit KI-Stärkepapier auf Peroxide getestet, über $MgSO_4$ getrocknet, filtriert und eingedampft. Man erhält 9,0 g (95 %) eines braunen Harzes.

$\eta^{160}$ = 780 mPa.s; Epoxidzahl = 4,97 Val/kg (95 %).

Beispiel 6: **Herstellung von Bisphenol-F-Diglycidylether**

In 250 ml DMSO werden 100,1 g (0,5 Mol) Bisphenol F und 133,9 g (1,75 Mol) Allylchlorid vorgelegt und innerhalb von einer Stunde 207,3 g (1,5 Mol) $K_2CO_3$ portionenweise zugegeben. Anschliessend wird auf 60 °C erwärmt und vier Stunden bei dieser Temperatur gerührt. Danach wird das Reaktionsgemisch abgekühlt, auf 750 ml Wasser gegossen und dreimal mit Dichlormethan extrahiert. Die vereinigten organischen Phasen werden dreimal mit Wasser gewaschen, mit Toluol versetzt und eingedampft. Es werden 132 g Diallylether von Bisphenol F als braune Flüssigkeit erhalten. Ein Teil davon wird am Dünnschichtverdampfer bei 185 °C/1,3 Pa destilliert.

14 g (0,05 Mol) des destillierten Produktes werden in einem Zweiphasensystem aus 50 ml Aceton, 50 ml Dichlormethan und 100 ml Phosphatpuffer pH = 7,0 gelöst und mit 0,66 g (2,5 mMol) 18-Krone-6 versetzt. Anschliessend werden innerhalb von dreieinhalb Stunden bei Raumtemperatur portionenweise 60 g (95 mMol) Oxone® zugegeben, wobei der pH-Wert mit 15 %iger NaOH konstant gehalten wird. Danach werden

die Phasen getrennt, die organische Phase mit 30 ml Aceton, 5 ml Dichlormethan, 100 ml Phosphatpuffer pH-Wert 7,0 und 0,66 g (2,5 mMol) 18-Krone-6 versetzt und nochmals 60 g (95 mMol) Oxone® innerhalb von dreieinhalb Stunden unter Konstanthaltung des pH-Werts zugegeben. Danach wird das Reaktionsgemisch mit Wasser verdünnt und dreimal mit Dichlormethan extrahiert. Die vereinigten organischen Phasen werden einmal mit 1N NaOH und viermal mit Wasser gewaschen, über $MgSO_4$ getrocknet, filtriert und eingedampft. Man erhält 12,6 g Bisphenol-F-Diglycidylether als klare braune Flüssigkeit mit einem Epoxidgehalt von 4,42 Val/kg (75%).

Beispiel 7: **Herstellung eines Glycidylethers eines Kresol-Formaldehyd-Novolaks**

100 g (0,7 Mol) eines Kresol-Formaldehyd-Novolaks ($M_n$ = 624; $M_w$ = 906) und 11,8 g (0,035 Mol) Tetrabutylammoniumhydrogensulfat werden in 160,7 g (2,1 Mol) Allylchlorid bei 30 °C gelöst und anschliessend portionenweise 140 g (3,5 Mol) NaOH innerhalb von einer Stunde zugegeben. Danach wird auf Rückfluss erhitzt und zwei Stunden bei dieser Temperatur gerührt. Das Reaktionsgemisch wird abgekühlt, mit Wasser versetzt und dreimal mit Methylethylketon extrahiert. Die vereinigten organischen Phasen werden dreimal mit 10 %iger $NH_4Cl$ Lösung und zweimal mit Wasser gewaschen, über $MgSO_4$ getrocknet, filtriert und eingedampft. Man erhält 126 g des Polyallylethers des Kresol-Formaldehyd-Novolaks als dunkelviolettes hochviskoses Harz. 50 g dieses Rohproduktes werden in möglichst wenig Essigsäureethylester gelöst und mit Hexan/Essigsäureethylester = 1:1 durch eine 20 cm lange Kieselgelsäule (Durchmesser 10 cm) filtriert. Man erhält 45 g eines hellgelben, hochviskosen Harzes.
19,1g (0,1 Mol) des gereinigten Produktes werden in einem Zweiphasensystem aus 50 ml Aceton, 50 ml Dichlormethan und 100 ml Phosphatpuffer pH = 7,0 gelöst und mit 0,66 g (2,5 mMol) 18-Krone-6 versetzt. Anschliessend werden innerhalb von dreieinhalb Stunden bei Raumtemperatur portionenweise 60 g (95 mMol) Oxone® zugegeben, wobei der pH-Wert mit 15 %iger NaOH konstant gehalten wird. Danach werden die Phasen getrennt, die organische Phase mit 20 ml Aceton, 10 ml Dichlormethan, 50 ml Phosphatpuffer pH-Wert 7,0 und 0,66 g (2,5 mMol) 18-Krone-6 versetzt und nochmals 60 g (95 mMol) Oxone® innerhalb von dreieinhalb Stunden unter Konstanthaltung des pH-Werts portionenweise zugegeben. Danach wird das Reaktionsgemisch mit Wasser verdünnt und dreimal mit Essigsäureethylester extrahiert. Die vereinigten organischen Phasen werden einmal mit 1N NaOH und dreimal mit Wasser gewaschen, mit Toluol versetzt und eingedampft. Man erhält 12 g des Polyglycidylethers des Kresol-Formaldehyd-Novolaks als braunen Feststoff mit einem Epoxidgehalt von 3,94 Val/kg (82%).

## Ansprüche

1. Verfahren zur Herstellung von Epoxidverbindungen aus aromatischen Allylethern mit mindestens einem direkt an den aromatischen Kern gebundenem Rest der Formel II

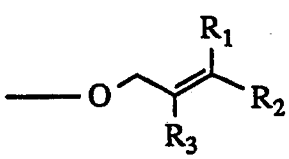 (II),

worin $R_1$, $R_2$ und $R_3$ unabhängig voneinander Wasserstoff oder $C_1$-$C_6$Alkyl bedeuten, dadurch gekennzeichnet, dass man das Ausgangsprodukt als solches oder in einem inerten Lösungsmittel gelöst mit einem Dioxiran epoxidiert, wobei auf eine Allylgruppe etwa 1 bis 20 Mole Dioxiran eingesetzt werden.

2. Verfahren gemäss Anspruch 1, worin der aromatische Allylether eine Verbindung der Formel III oder eine Verbindung, die im wesentlichen aus wiederkehrenden Struktureinheiten der Formeln IV oder V besteht, ist

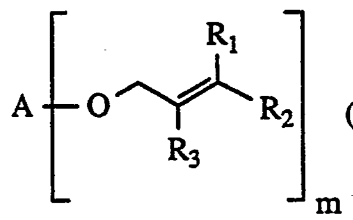 (III), 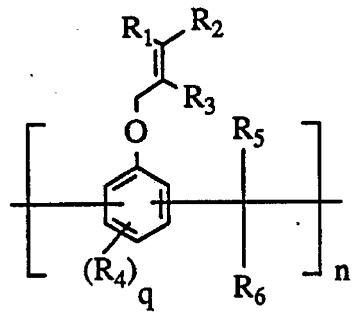 (IV),

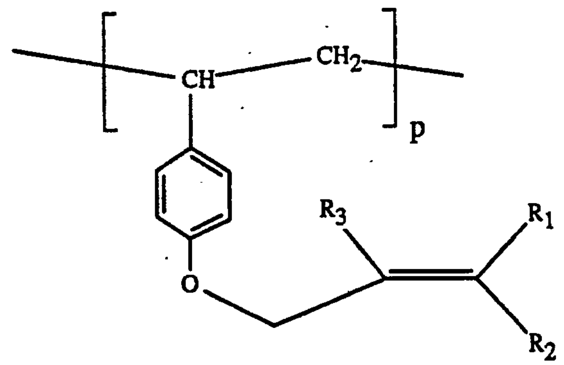 (V),

worin m 1,2,3 oder 4ist, n eine ganze Zahl von 2 bis 12 ist, p eine ganze Zahl von 2 bis 100 bedeutet, q 0,1,2 oder 3 ist, $R_1$, $R_2$ und $R_3$ die in Anspruch 1 definierte Bedeutung besitzen, $R_4$ $C_1$-$C_6$Alkyl oder Halogen bedeutet, $R_5$ und $R_6$ unabhängig voneinander Wasserstoff oder $C_1$-$C_6$Alkyl sind und A einen m-wertigen aromatischen Rest eines Phenols nach dem Entfernen der Hydroxylgruppen darstellt.

3. Verfahren gemäss Anspruch 2, worin $R_1$ und $R_2$ Wasserstoff bedeuten und $R_3$ Methyl oder insbesondere Wasserstoff ist.

4. Verfahren gemäss Anspruch 2, worin der aromatische Allylether eine Verbindung der Formeln III, IV oder V ist, worin m 2 ist, n 3 bis 6 bedeutet, p 5 bis 40 darstellt, q 0 ist, A ein zweikerniger Rest eines Bisphenols ist und $R_1$, $R_2$ und $R_3$ Wasserstoff bedeuten.

5. Verfahren gemäss Anspruch 1, worin der aromatische Allylether als solcher oder in einem unter den Reaktionsbedingungen inerten Lösungsmittel gelöst vorgelegt wird und eine Dioxiranlösung in einem Keton zugegeben wird, wobei pro Mol zu epoxidierender Doppelbindung etwa ein bis zwanzig Mole Dioxiran verwendet werden.

6. Verfahren gemäss Anspruch 1, worin der aromatische Allylether als solcher oder in einem unter den Reaktionsbedingungen inerten Lösungsmittel gelöst zusammen mit einem Phasentransferkatalysator und in Kombination mit einem Keton und Wasser vorgelegt wird, so dass ein Zweiphasensystem entsteht, wobei die wässrige Phase einen Puffer enthält, mit dem ein pH-Wert im Bereich von etwa 6 bis 10 eingestellt wird, und anschliessend ein Peroxomonosulfat zugegeben wird, wobei die Menge an Peroxomonosulfat etwa ein bis zehn Mol pro Mol zu epoxidierender Doppelbindung beträgt.

7. Verfahren gemäss Anspruch 6, worin als Phasentransferkatalysator ein Kronenether oder ein quaternäres Ammonium- oder Phosphoniumsalz und als Puffer ein Phosphatpuffer verwendet wird, mit dem ein pH-Wert von etwa 7 eingestellt wird.

8. Verfahren gemäss Anspruch 6, worin der aromatische Allylether gelöst in einem inerten Lösungsmittel zusammen mit einem Kronenether oder einem quaternären Ammoniumsalz als Phasentransferkatalysator vorgelegt wird und eine auf einen pH-Wert von 6,5 bis 7,5 eingestellte wässrige Acetonphase verwendet wird, wobei das Peroxomonosulfat fest zugegeben wird und der pH-Wert durch Zugabe einer wässrigen Lösung einer Base, insbesondere Natronlauge, konstant gehalten wird.

9. Verfahren gemäss Anspruch 1, worin die Reaktionstemperaturen zwischen 0 und 30° C liegen.

10. Verfahren gemäss Anspruch 1, worin als Epoxidierungsreagenz Bis-trifluormethyldioxiran oder insbesondere Dimethyldioxiran verwendet wird.

11. Verfahren gemäss Anspruch 1, worin der aromatische Allylether vor der Epoxidierungsstufe durch Chromatographie oder durch Destillation gereinigt wird.

12. Verfahren zur Herstellung von gehärteten Produkten, dadurch gekennzeichnet, dass man die nach dem Verfahren gemäss Anspruch 1 erhältlichen Epoxidharze durch Zugabe eines an sich bekannten Härtungsmittels und gegebenenfalls durch Erhitzen härtet.